# EUROPÄISCHE PATENTSCHRIFT

(11) **EP 1 804 846 B1**
(45) Veröffentlichungstag und Bekanntmachung des Hinweises auf die Patenterteilung: **06.02.2008**
(21) Anmeldenummer: 05802890.3
(22) Anmeldetag: 27.10.2005
(51) Int. Cl.: A61L 29/16, A61M 25/00

(54) **VENENVERWEILKANÜLE**
INDWELLING VENOUS CANNULA
CANULE VEINEUSE A DEMEURE

(30) Priorität: 28.10.2004 DE 102004052535
(43) Veröffentlichungstag der Anmeldung: 11.07.2007
(73) Patentinhaber: Universität Duisburg-Essen, 45141 Essen (DE)
(72) Erfinder: VON KNOCH, Marius, 45239 Essen (DE)
(74) Vertreter: Gesthuysen, von Rohr & Eggert
(86) Internationale Anmeldenummer: PCT/EP2005/011513
(87) Internationale Veröffentlichungsnummer: WO 2006/045608

(56) Entgegenhaltungen:
- DE-A1- 4 041 720
- US-A- 4 253 463
- US-A- 5 468 562

## Beschreibung

Die vorliegende Erfindung betrifft eine Venenverweilkanüle gemäß dem Oberbegriff des Anspruchs 1.

Übliche Venenverweilkanülen weisen eine flexible Kunststoffkanüle zur Verlegung durch die Haut in eine Vene eines Patienten auf. Problematisch ist, daß mit der Zeit Infektionen auftreten, die durch krankheitserregende Organismen, insbesondere Bakterien, verursacht werden, die von der Haut des Patienten entlang der Kunststoffkanüle "aufsteigen" bzw. eindringen können.

Die US 5,468,562 A offenbart eine Kunststoffkanüle, beispielsweise für ein intravenöses Infusionssystem, die gegen Infektionen vollständig metallisch beschichtet ist. Tatsächlich werden derartige Kunststoffkanülen nicht oder allenfalls sehr wenig eingesetzt. Problematisch ist, daß sich Beschichtungen lösen können, und/oder zu einer unerwünschten Versteifung der Kunststoffkanüle führen. Des weiteren erfordert eine derartige Beschichtung eine erneute klinische Zulassung der Venenverweilkanüle, um beispielsweise ausschließen zu können, daß Stenosen in der Vene durch die Beschichtung verursacht werden.

Die DE 40 41 720 A1 schlägt eine Venenverweilkanüle vor, wobei der in den Körper eindringende Teil aus einem bei Kontakt mit Flüssigkeiten Metallionen abgebenden, oligodynamisch wirkenden Metall oder einer entsprechenden Metallegierung oder aus Edelstahl besteht. Hier soll zur Vermeidung von Infektionen anstelle der üblichen Kunststoffkanüle ein Metallröhrchen eingesetzt werden. Nachteilig ist die geringere Flexibilität eines Metallröhrchens gegenüber einer Kunststoffkanüle, so daß potentiell eine höhere Verletzungsgefahr besteht und ein geringerer Tragekomfort zu erwarten ist.

Die US 4,266,999 A schlägt eine Kanüle bzw. einen Katheter vor, wobei eine elastische Beschichtung oberflächenbehandelt und umgeschlagen wird, um ein Einwachsen der Haut zu ermöglichen und so eine Barriere gegen Infektionen zu bilden. Das Einwachsen dauert jedoch eine gewisse Zeit und kann Infektionen nicht sicher verhindern. Zudem ist diese Lösung sehr aufwendig.

Die US 4,253,463 A offenbart eine medizinische intravaskuläre Einrichtung mit einer Kunststoffkanüle, die mit einer metallischen Beschichtung versehen ist.

Der vorliegenden Erfindung liegt die Aufgabe zugrunde, eine Venenverweilkanüle anzugeben, die bei einfachem, kostengünstigem Aufbau einen wirksamen Schutz gegen Infektionen ermöglicht.

Die obige Aufgabe wird durch eine Venenverweilkanüle gemäß Anspruch 1 gelöst. Vorteilhafte Weiterbildungen sind Gegenstand der Unteransprüche.

Eine grundlegende Idee der vorliegenden Erfindung liegt darin, eine antibakteriell und/oder oligodynamisch wirkende - also gegen Infektionen wirkende - Hülse vorzusehen, die die Kunststoffkanüle ausschließlich im spitzenfernen Hautdurchtrittsbereich umgibt. Vorzugsweise besteht die Hülse aus Titan oder einer Titanlegierung oder aus einem sonstigen geeigneten Metall. Jedoch sind auch antibakteriell wirkende Kunststoffe, wie Polylactid, einsetzbar.

Die Hülse führt zu einem wirksamen Schutz gegen Infektionen ohne die Nachteile des Standes der Technik.

Die vorschlagsgemäße Venenverweilkanüle ist sehr einfach und kostengünstig herstellbar, da die Hülse zusätzlich bei bekannten Konstruktionen angeordnet oder gegebenenfalls sogar nachgerüstet werden kann.

Die vorgesehene Hülse ist nur im Hautdurchtrittsbereich angeordnet und führt dazu, daß die vorschlagsgemäße Venenverweilkanüle einer neuen Zulassung nicht bedarf.

Die Hülse führt lediglich zu einer Versteifung im Hautdurchtrittsbereich, so daß der Tragekomfort - also die Flexibilität der Kunststoffkanüle in den sonstigen Bereichen und insbesondere in dem in der Vene liegenden Bereichnicht verschlechtert wird.

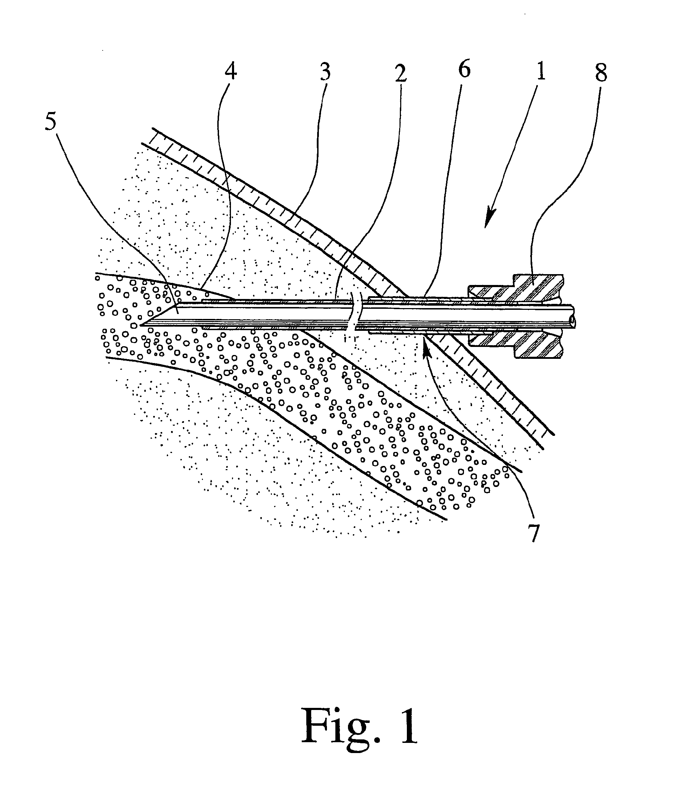

Weitere Vorteile, Merkmale, Eigenschaften und Aspekte der vorliegenden Erfindung ergeben sich aus der nachfolgenden Beschreibung eines bevorzugten Ausführungsbeispiels anhand der Zeichnung. Die einzige Figur zeigt:
eine schematische, nicht maßstabsgerechte Schnittansicht einer vorschlagsgemäßen Venenverweilkanüle beim Einführen in eine Vene.

Die einzige Figur zeigt eine vorschlagsgemäße Venenverweilkanüle 1 in einer nicht maßstabsgerechten Schnittdarstellung beim Einführen. Die Venenverweilkanüle 1 weist eine flexible Kunststoffkanüle 2 zur Verlegung durch die Haut 3 in eine Vene 4 eines nicht näher dargestellten Patienten auf.

Das Einführen erfolgt insbesondere mittels einer inneren, herausziehbaren Punktiernadel oder -kanüle 5, wie dargestellt. Nach dem Einführen wird dann die Punktiernadel bzw. -kanüle 5 aus der Kunststoffkanüle 2 herausgezogen. Die Kunststoffkanüle 2 ist dann aufgrund ihrer hohen Flexibilität relativ beweglich, so daß sich ein guter Tragekomfort für den Patienten ergibt.

Die Venenverweilkanüle 1 weist vorschlagsgemäß eine antibakteriell und/oder oligodynamisch wirkende Hülse 6 auf, die die Kunststoffkanüle 2 umgibt und zumindest im wesentlichen ausschließlich im spitzenfernen Hautdurchtrittsbereich 7 angeordnet ist. Insbesondere ist die Hülse 6 auf die durchgehende Kunststoffkanüle aufgeschoben. Die Hülse 6 erstreckt sich über den Austrittsbereich der Kunststoffkanüle 2 aus der Haut 3 hinaus, und zwar vorzugsweise bis zu einem sich an die Kunststoffkanüle 2 anschließenden Anschlußstück 8.

Dementsprechend bildet die vorschlagsgemäße Hülse 6 eine wirksame Infektionsbarriere, da infektionserregende Organismen, insbesondere Bakterien, nicht mehr entlang der Kunststoffkanüle 2 von außen durch die Haut 3 hindurch in den Körper hineinwandern können, sondern jetzt auf die Außenfläche der Hülse 6 angewiesen sind, die aufgrund ihrer antibakteriellen bzw. oligodynamischen Wirkung das genannte Hineinwandern verhindert.

Die Kunststoffkanüle 2 endet beim Darstellungsbeispiel in dem Anschlußstück 8. Beispielsweise ist das Anschlußstück 8 angeklemmt, angeklebt und/oder angespritzt.

Die Hülse 6 besteht vorzugsweise aus Metall, insbesondere Titan oder einer Titanlegierung. Metall wirkt nämlich wesentlich antibakterieller als die üblicherweise für die Kanüle 2 eingesetzten Kunststoffe. Jedoch kann die Hülse 6 bedarfsweise auch aus antibakteriell wirkendem Kunststoff, wie Polylactid oder dergleichen, bestehen.

Vorzugsweise besteht die Hülse 6 aus oligodynamisch wirkendem Material, also aus Material, das aufgrund der Abgabe von Metallionen in Gegenwart von Flüssigkeit seine Wirkung gegen Infektionen hervorrufende Organismen, insbesondere Bakterien oder dergleichen, entfaltet.

Die Hülse 6 ist vorzugsweise im wesentlichen nur in dem Hautdurchtrittsbereich 7 angeordnet. Dementsprechend ist die Hülse 6 verhältnismäßig kurz ausgebildet. Vorzugsweise beträgt ihre Länge weniger als 12 mm, insbesondere im wesentlichen nur 4 bis 8 mm.

Die Hülse 6 ist bedarfsweise auf die Kunststoffkanüle 2 aufgeschrumpft, aufgeklemmt und/oder mit dieser verklebt.

Alternativ oder zusätzlich ist die Hülse 6 mit dem Anschlußstück 8 verbunden oder davon gehalten. Insbesondere ist die Hülse 6 mit einem Endbereich in das Anschlußstück 8 eingespritzt oder mit diesem verklebt.

Wahlweise kann die Hülse 6 von dem Anschlußstück 8 auch klemmend gehalten sein, insbesondere wenn sich die Hülse 6 in einen konusartigen oder ringförmigen Spalt zwischen der Kunststoffkanüle 2 und dem Anschlußstück 8 erstreckt, wie in der Figur angedeutet.

An ihrem anderen Ende, also an ihrem vorderen, in die Haut 3 einzuführenden Ende ist die Hülse 6 vorzugsweise konisch verjüngt, um ein einfaches Einführen zu ermöglichen.

Alternativ oder zusätzlich ist die Wandungsdicke der Hülse 6 möglichst gering und beträgt beispielsweise höchstens 0,5 mm, ganz bevorzugt nur etwa 0,3 bis 0,1 mm.

Die Hülse 6 ist vorzugsweise intransparent ausgebildet und weist insbesondere eine andere Farbe als die Kunststoffkanüle 2 auf, so daß die Hülse 6 deutlich sichtbar und erkennbar ist. Auf diese Weise ist für Benutzer von Venenverweilkanülen 1 unmittelbar ersichtlich, ob es sich um die vorschlagsgemäße Venenverweilkanüle 1 mit dem durch die Hülse 6 gebildeten Infektionsschutz handelt.

Gemäß einer nicht dargestellten Ausführungsvariante kann die Hülse 6 auch durch eine entsprechende, im wesentlichen nur im Hautdurchtrittsbereich 7 angeordnete, also partielle Beschichtung der Kunststoffkanüle 2 gebildet sein. Mittels der partiellen Beschichtung sind dann die gleichen oder entsprechende Vorteile wie durch die Hülse 6 erreichbar.

Bedarfsweise kann die Beschichtung verhältnismäßig starr ausgebildet sein bzw. zu einer starken Versteifung der Kunststoffkanüle 2 im Hautdurchtrittsbereich 7 entsprechend der Hülse 6 führen. Dies gestattet eine sehr dicke und/oder haltbare Ausbildung der Beschichtung, so daß ein ungewolltes Ablösen mit Sicherheit ausgeschlossen werden kann.

Die Kunststoffkanüle 2 besteht vorzugsweise aus dem üblicherweise verwendeten Kunststoff. Jedoch kann grundsätzlich auch ein sonstiges geeignetes Material eingesetzt werden. Entsprechend ist der Begriff "Kunststoff" in einem weiten Sinn zu verstehen.

## Patentansprüche

1. Venenverweilkanüle (1) mit einer flexiblen Kunststoffkanüle (2) zur Verlegung durch die Haut (3) in eine Vene (4) eines Patienten, insbesondere mittels einer inneren, herausziehbaren Punktiernadel oder -kanüle (5), und mit einer antibakteriell und/oder oligodynamisch wirkenden Hülse (6),
**dadurch gekennzeichnet,**
**daß** die Hülse (6) die Kunststoffkanüle (2) ausschließlich im spitzenfernen Hautdurchtrittsbereich (7) umgibt.

2. Venenverweilkanüle nach Anspruch 1, **dadurch gekennzeichnet, daß** die Hülse (6) aus Metall, insbesondere Titan oder einer Titanlegierung, oder aus antibakteriell wirkendem Kunststoff, wie Polylactid, besteht.

3. Venenverweilkanüle nach einem der voranstehenden Ansprüche, **dadurch gekennzeichnet, daß** die Hülse (6) aus oligodynamisch wirkendem Material besteht und/oder daß sich die Hülse (6) über den Austrittsbereich der Kunststoffkanüle (2) aus der Haut (3) hinaus erstreckt.

4. Venenverweilkanüle nach einem der voranstehenden Ansprüche, **dadurch gekennzeichnet, daß** die Venenverweilkanüle (1) ein sich an die Kunststoffkanüle (2) anschließendes Anschlußstück (8) aufweist und daß sich die Hülse (6) bis zu dem Anschlußstück (8) erstreckt.

5. Venenverweilkanüle nach Anspruch 4, **dadurch gekennzeichnet, daß** die Hülse (6) mit dem Anschlußstück (8) verbunden oder davon gehalten ist, insbesondere wobei die Hülse (6) mit einem Endbereich in das Anschlußstück (8) eingespritzt ist.

6. Venenverweilkanüle nach Anspruch 5, **dadurch gekennzeichnet, daß** die Hülse (6) von dem Anschlußstück (8) klemmend gehalten ist und/oder daß die Hülse (6) mit dem Anschlußstück (8) verklebt ist.

7. Venenverweilkanüle nach einem der voranstehenden Ansprüche, **dadurch gekennzeichnet, daß** die Hülse (6) eine Länge von weniger als 12 mm, insbesondere im wesentlichen von 4 bis 8 mm, aufweist und/oder daß die Hülse (6) auf die Kunststoffkanüle (2) aufgeschrumpft oder aufgeklemmt ist.

8. Venenverweilkanüle nach einem der voranstehenden Ansprüche, **dadurch gekennzeichnet, daß** die Hülse (6) auf die Kunststoffkanüle (2) aufgeschoben ist, und/oder daß die Hülse (6) mit der Kunststoffkanüle (2) verklebt ist.

9. Venenverweilkanüle nach einem der voranstehenden Ansprüche, **dadurch gekennzeichnet, daß** die Hülse (6) sichtbar ist, insbesondere eine andere Farbe als die Kunststoffkanüle (2) aufweist, und/oder daß die Hülse (6) an ihrem vorderen, in die Haut (3) einzuführenden Ende konisch verjüngt ist.

## Claims

1. An indwelling venous cannula (1) with a flexible plastic cannula (2) for passing through the skin (3) into a vein (4) of a patient, particularly by means of an inner, removable puncture needle or cannula (5), and with a sheath (6) which acts in an antibacterial and/or oligodynamic manner,
**characterized in**
**that** the sheath (6) surrounds the plastic cannula (2) exclusively in the penetration region (7) of the skin away from the tip.

2. The indwelling venous cannula according to Claim 1, **characterized in that** the sheath (6) consists of metal, in particular titanium or a titanium alloy, or of plastic which acts in an antibacterial manner, such as polylactid.

3. The indwelling venous cannula according to any of the preceding claims, **characterized in that** the sheath (6) consists of material which acts in an oligodynamic manner and/or that the sheath (6) extends over the outlet region of the plastic cannula (2) out of the skin (3).

4. The indwelling venous cannula according to any of the preceding claims, **characterized in that** the indwelling venous cannula (1) has a connection piece (8) adjoining the plastic cannula (2) and that the sheath (6) extends up to the connection piece (8).

5. The indwelling venous cannula according to Claim 4, **characterized in that** the sheath (6) is connected with the connection piece (8) or is held thereby, in particular with the sheath (6) being injected with an end region into the connection piece (8).

6. The indwelling venous cannula according to Claim 5, **characterized in that** the sheath (6) is held by the connection piece (8) in a clamping manner and/or that the sheath (6) is glued to the connection piece (8).

7. The indwelling venous cannula according to any of the preceding claims, **characterized in that** the sheath (6) has a length of less than 12 mm, in particular substantially from 4 to 8 mm, and/or that the sheath (6) is shrunken or clamped onto the plastic cannula (2).

8. The indwelling venous cannula according to any of the preceding claims, **characterized in that** the sheath (6) is pushed onto the plastic cannula (2), and/or that the sheath (6) is glued to the plastic cannula (2).

9. The indwelling venous cannula according to any of the preceding claims, **characterized in that** the sheath (6) is visible, in particular has a different colour from the plastic cannula (2), and/or that the sheath (6) is tapered conically at its front end which is to be introduced into the skin (3).

## Revendications

1. Cathéter (1) avec une canule flexible en matière plastique (2) destinée à être posée à travers la peau (3) dans une veine (4) d'un patient, notamment au moyen d'une aiguille ou canule de ponction (5) extractible et avec une gaine (6) à effet antibactérien et/ou oligodynamique,
**caractérise en ce que,**
la gaine (6) entoure la canule en matière plastique (2) uniquement dans la zone de traversée de la peau (7) éloignée de la pointe.

2. Cathéter selon la revendication 1, **caractérisé en ce que** la gaine (6) est en métal, notamment en titane ou en un alliage de titane, ou en une matière plastique à effet antibactérien, comme le polyactide.

3. Cathéter selon l'une quelconque des revendications précédentes, **caractérisé en ce que** la gaine (6) consiste dans une matière à effet oligodynamique et/ou **en ce que** la gaine (6) s'étend sur la zone de sortie de la canule en matière plastique (2) hors de la peau (3).

4. Cathéter selon l'une quelconque des revendications précédentes, **caractérisé en ce que** le cathéter (1) comporte une pièce de raccordement (8) se raccordant sur la canule en matière plastique (2) et **en ce que** la gaine (6) s'étend jusqu'à la pièce de raccordement (8).

5. Cathéter selon la revendication 4, **caractérisé en ce que** la gaine (6) est reliée avec la pièce de raccordement (8) ou maintenue par cette dernière, la gaine (6) étant notamment injectée dans la pièce de raccordement (8), par une zone d'extrémité.

6. Cathéter selon la revendication 5, **caractérisé en ce que** la gaine (6) est maintenue par serrage par la pièce de raccordement (8) et/ou **en ce que** la gaine (6) est collée sur la pièce de raccordement (8).

7. Cathéter selon l'une quelconque des revendications précédentes, **caractérisé en ce que** la gaine (6) a une longueur inférieure à 12 mm, notamment sensiblement de 4 à 8 mm, et/ou **en ce que** la gaine (6) est thermoformée ou serrée sur la canule en matière plastique (2).

8. Cathéter selon l'une quelconque des revendications précédentes, **caractérisé en ce que** la gaine (6) est emboîtée sur la canule en matière plastique (2) et/ou **en ce que** la gaine (6) est collée sur la canule en matière plastique (2).

9. Cathéter selon l'une quelconque des revendications précédentes, **caractérisé en ce que** la gaine (6) est visible, notamment elle présente un autre teinte que la canule en matière plastique (2) et/ou **en ce que** la gaine (6) se rétrécit sous forme conique, sur son extrémité antérieure, destinée à être introduite dans la peau (3).
